# EUROPÄISCHE PATENTSCHRIFT

(11) **EP 1 192 964 B1**
(45) Veröffentlichungstag und Bekanntmachung des Hinweises auf die Patenterteilung: **11.12.2002**
(21) Anmeldenummer: 00121060.8
(22) Anmeldetag: 27.09.2000
(51) Int. Cl.: A61M 5/31

(54) **Spritze mit einem ein fluides Medium aufnehmenden Zylinder und einer Verschlusskappe**
Syringe with fluid reservoir and closure cap
Seringue avec réservoir cylindrique et capuchon de fermeture

(43) Veröffentlichungstag der Anmeldung: 03.04.2002
(73) Patentinhaber: Schering Aktiengesellschaft, 13353 Berlin (DE)
(72) Erfinder: Witowski, Norbert, 38302 Wolfenbüttel (DE)
(74) Vertreter: Prüfer, Lutz H., Dipl.-Phys.

(56) Entgegenhaltungen:
- EP-A- 0 685 237
- EP-A- 0 723 784
- WO-A-95/01812
- DE-C- 854 617
- US-A- 2 922 419
- US-A- 4 568 336

## Beschreibung

Die Erfindung betrifft eine Spritze nach dem Oberbegriff des Patentanspruches 1.

Eine andere Spritze ist aus der EP 0 723 784 A bekannt. Der Zylinder weist eine sich in Umfangsrichtung erstreckende Nase mit einer dem Ausgang zugewandten abgeschrägten Flanke und einer nahezu zur Umfangsrichtung senkrechten rückwärtigen Schulter auf. Die aufzusetzende Kappe weist auf ihrer Innenseite sich in Umfangsrichtung erstreckende als Hinterschneidungen ausgebildete Abschnitte auf, die beim Aufschieben der Kappe der hervorstehende Abschnitt des Zylinders einrastet. Da der äußere Durchmesser des hervorstehenden Abschnittes des Zylinders größer als der Innendurchmesser der Kappe ist, ist eine erhebliche Druckkraft beim Aufbringen der Kappe aufzuwenden. Das gilt um so mehr, als das hervorstehende Element relativ groß sein muß, damit Abweichungen der Behältnisabmessungen ausgeglichen werden können.

Aus der EP 0 685 237 A ist eine Spritze nach dem Oberbegriff des Anspruches 1 oder des Anspruches 2 zu entnehmen. Insbesondere sind bei dieser Spritze Dehnungsabschnitte durch Schlitze realisiert, die bis zum offenen Ende der Verschlußkappe geführt sind und daher an dem offenen Ende der Verschlußkappe offen sind. Das ermöglicht ein Aufbiegen des offenen Endes der Verschlußkappe.

Aus der DE 854 617 C ist ein Flaschenverschluß zu entnehmen, der leicht auf Flaschen angebracht und abgenommen werden kann, so daß er wiederholt verwendet werden kann. Der Verschluß weist dazu Ausschnitte in der Seitenwand auf.

Aufgabe der Erfindung ist es, eine verbesserte Spritze der eingangs beschriebenen Art zu schaffen.

Diese Aufgabe wird durch die in Patentanspruch 1 oder 2 gekennzeichnete Spritze gelöst.

Durch diese Ausbildung wird erreicht, daß der Aufpreßdruck zum Aufbringen der Verschlußkappe geringer sein kann, was wiederum zur Folge hat, daß auch die Zahl der Beschädigungen der Behältnisse beim Verschließen reduziert wird.

Weiterbildungen der Erfindung sind in den Unteransprüchen gekennzeichnet.

Weitere Merkmale und Zweckmäßigkeiten der Erfindung ergeben sich aus der Beschreibung von Ausführungsbeispielen anhand er Figuren. Von den Figuren zeigen:
- Fig.1: einen Schnitt durch das ausgangsseitige Ende eines Spritzenzylinders und durch die aufzusetzende Verschlußkappe und
- Fig.2: eine zweite Ausführungsform des ausgangsseitigen Endes des Spritzenzylinders.

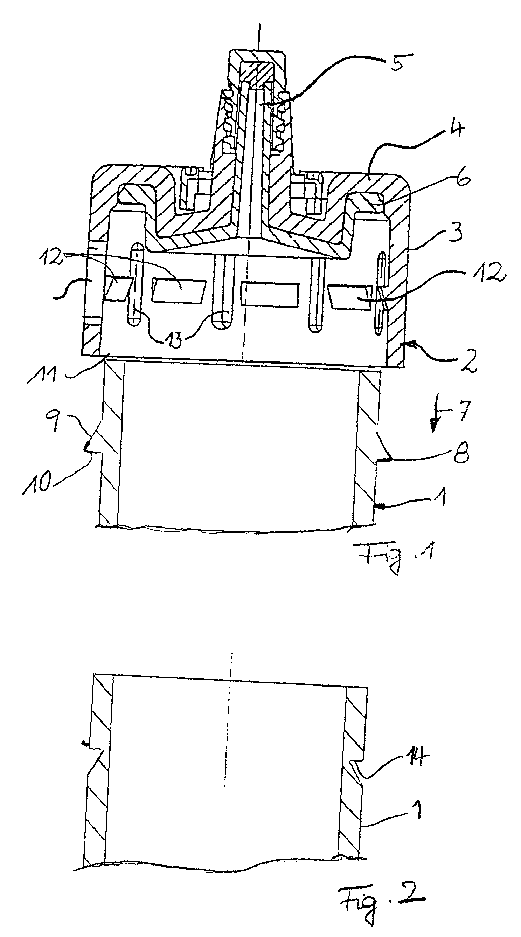

In Fig. 1 ist das ausgangsseitige Ende eines Zylinders 1 gezeigt, auf welches zum ausgangsseitigen Verschließen eine Verschlußkappe 2 aufgesetzt wird. Der Zylinder 1 weist in bekannter Weise auf seiner Eingangsseite einen Verschluß und im Inneren einen verschiebbaren Kolben zum Ausdrücken des in dem Zylinder aufzunehmenden Mediums auf. Der Zylinder 1 ist als Glaszylinder ausgebildet und die Verschlußkappe 2 besteht vorzugsweise aus einem Chlorbutylkautschuk.

Die Verschlußkappe 2 ist becherförmig ausgebildet und weist einen zylinderförmigen Wandabschnitt 3 auf, der an dem dem Zylinder zugewandten Ende offen ist und auf dem gegenüberliegenden Ende einen Deckel 4 aufweist, wobei der Deckel in bekannter Weise einen verschließbaren Ausgang 5 besitzt. Im Inneren des Deckels ist eine Dichtung 6 vorgesehen. Die Verschlußkappe 2 wird zum Verschließen des Spritzenkörpers in Richtung des Pfeiles 7 auf das ausgangsseitige Ende des Zylinders 1 aufgeschoben.

Der Zylinder 1 weist in einem vorbestimmten Abstand von seinem ausgangsseitigen Ende ein sich über den gesamten Umfang erstreckendes erstes Rastelement 8 auf, welches in seinem Querschnitt nach Art eines Sägezahnes ausgebildet ist und auf seiner dem ausgangsseitigen Ende zugewandten Schulter 9 rampenartig abgeschrägt ist, während die dem ausgangsseitigen Ende abgewandte zweite Schulter 10 sich nahezu senkrecht zur Zylinderoberfläche erstreckt.

In einem vorbestimmten Abstand vom offenen Ende 11 sind auf der Innenwand eine Mehrzahl von in Umfangsrichtung gegeneinander versetzte zweite Rastelemente 12 vorgesehen. Diese sind ebenfalls im Querschnitt etwa sägezahnförmig ausgebildet und weisen auf ihrer dem offenen Ende 11 zugewandten Seite eine wiederum abgeschrägte Schulter und auf ihrer dem offenen Ende 11 abgewandten Seite eine Schulter auf, die nahezu senkrecht zur Wandung des Wandabschnittes verläuft. Die zweiten Schultern liegen in einer senkrecht zur Symmetrieachse der Verschlußkappe verlaufenden Ebene. Der Abstand dieser Ebene von dem offenen Ende 11 bzw. vom Grund der Dichtung 6 her ist so gewählt, daß die Verschlußkappe gerade auf das ausgangsseitige Ende des Zylinders 1 so aufschiebbar ist, daß bei Anliegen des freien Randes des Zylinders an der Dichtung 6 die zweiten Rastelemente 12 über das erste Rastelement 8 hinüber geglitten sind und die beiden Elemente mit ihren nahezu senkrechten Schultern verriegelnd in Kontakt gelangen.

Wie aus Fig. 1 ersichtlich ist, ist zwischen jeweils zwei benachbarten zweiten Rastelementen 12 ein sich parallel zur Symmetrieachse erstreckender Längsschlitz 13 in der Wandung der Verschlußkappe vorgesehen. Dieser ist symmetrisch zwischen jeweils zwei benachbarten Rastelementen angeordnet und erstreckt sich sowohl nach oben zum geschlossenen Boden der Verschlußkappe als auch nach unten zum offenen Ende 11 hin über die Länge der Rastelemente 12 hinaus.

Bevorzugt sind in Umfangsrichtung gegeneinander versetzt wenigstens sechs zweite Rastelemente vorgesehen. Als ganz besonders vorteilhaft ist eine Ausführungsfrom mit einer Anzahl von acht bis zehn und entsprechend jeweils dazwischen angeordneten Längsschlitzen 13.

Die in Fig. 2 gezeigte Ausführungsform unterschiedet sich von der zuerst beschriebenen lediglich dadurch, daß anstelle des ersten Rastelementes 8 eine ringförmige Hinterschneidung 14 vorgesehen ist, die so ausgebildet ist, daß die zweiten Rastelemente 12 beim Aufschieben der Verschlußkappe 2 in die Hinterschneidung zur Verriegelung eingreifen.

Die Längsschlitze 13 dienen als Dehnungsabschnitte und erleichtern das Aufschieben der Verschlußkappe auf das Ende des Zylinders. Auch die oben diskutierte Anzahl der umfangsrichtungsmäßig angeordneten zweiten Rastelemente 12 trägt dazu bei, daß in Verbindung mit den Längsschlitzen ein Aufschieben mit weniger Druck möglich wird.

In dem oben beschriebenen Ausführungsbeispiel sind die durch die Längsschlitze 13 gebildeten Dehnungsabschnitte als durch die ganze Wand sich erstreckende Ausnehmungen ausgebildet. Alternativ kann auch vorgesehen sein, daß die Dehnungsstreifen nur als nicht durch die gesamte Wand hindurchgehende Ausnehmungen ausgebildet sind. In beiden Fällen wir die axiale Länge und die Breite der Dehnungsstreifen in Abhängigkeit von dem die Verschlußkappe bildenden Material und dessen Elastizitätseigenschaften gewählt. Der Zylinder selbst ist dabei bevorzugt aus Glas gebildet.

Die Anforderungen an die Materialien von Glaskolben, eingangsseitigem Verschluß, ausgangsseitiger Verschlußkappe und Druckkolben, die zusammen eine Glaskartusche bilden, sind hoch, da sie sowohl in nicht montiertem Zustand als auch als montierte Spritze der Sterilisation bei etwa 120° C über einen längeren Zeitraum von beispielsweise 30 Minuten mit überhitztem Wasser unterworfen werden.

## Patentansprüche

1. Spritze mit einem ein fluides Medium aufnehmenden Zylinder (1) und einer ausgangsseitig aufgesetzten Verschlußkappe (2) mit einem einen verschließaren Ausgang (5) aufweisen den Deckel (4), wobei der Zylinder (1) an dem in die Kappe (2) einführbaren Abschnitt außen ein erstes Rastelement (8) und die Kappe (2) an dem entsprechenden Abschnitt innen damit zusammenwirkende zweite Rastelemente (12) aufweist,
die Kappe (2) eine Mehrzahl von in Umfangsrichtung angeordneten zweiten Rastelementen (12) aufweist,
zwischen einander benachbarten zwei Rastelementen (12) in der Wandung der Kappe (2) ein in axialer Richtung der Verschlußkappe (2) länglicher Dehnungsabschnit (13) vorgesehen ist und
der Dehnungsabschnit (13) sich in der axialen Richtung der Kappe (2) gesehen über die in axialer Richtung gesehenen Ausdehnungen der zweiten Rastelemente (12) erstreckt,
**dadurch gekennzeichnet,**
**daß** das zum offenenen Ende (11) der Verschlußkappe (2) hin gerichtete Ende des Dehnungsabschnittes (13) einen Abstand von dem offenen Ende (11) der Verschlußkappe (2) aufweist und
**daß** das erste Rastelement (8) als über die Zylinderoberfläche hervorstehende Nase ausgebildet ist, die auf einem dem ausgangsseitigen Ende zugewandten Abschnitt (9) abgeschrägt und auf dem dem ausgangsseitigen Ende abgewandten zweiten Abschnitt eine zum Zylindermantel nahezu senkrecht verlaufende Schulter (10) aufweist.

2. Spritze mit einem ein fluides Medium aufnehmenden Zylinder (1) und einer ausgangsseitig aufgesetzten Verschlußkappe (2) mit einem einen verschließaren Ausgang (5) aufweisen den Deckel (4), wobei der Zylinder (1) an dem in die Kappe (2) einführbaren Abschnitt außen ein erstes Rastelement (8) und die Kappe (2) an dem entsprechenden Abschnitt innen damit zusammenwirkende zweite Rastelemente (12) aufweist,
die Kappe (2) eine Mehrzahl von in Umfangsrichtung angeordneten zweiten Rastelementen (12) aufweist,
zwischen einander benachbarten zwei Rastelementen (12) in der Wandung der Kappe (2) ein in axialer Richtung der Verschlußkappe (2) länglicher Dehnungsabschnitt (13) vorgesehen ist und
der Dehnungsabschnit (13) sich in der axialen Richtung der Kappe (2) gesehen über die in axialer Richtung gesehenen Ausdehnungen der zweiten Rastelemente (12) erstreckt,
**dadurch gekennzeichnet,**
**daß** das zum offenenen Ende (11) der Verschlußkappe (2) hin gerichtete Ende des Dehnungsabschnittes (13) einen Abstand von dem offenen Ende (11) der Verschlußkappe (2) aufweist und
**daß** das erste Rastelement (8) als eine sich in Umfangsrichtung erstreckende Ausnehmung (14) ausgebildet ist, die auf einem dem ausgangsseitigen Ende abgewandten Abschnitt abgeschrägt und auf dem dem ausgangsseitigen Ende zugewandten zweiten Abschnitt eine zum Zylindermantel nahezu senkrecht Verlaufende Schulter aufweist.

3. Spritze nach Anspruch 1 oder 2, bei der der Dehnungsabschnitt (13) als verdünnter Wandabschnitt ausgebildet ist.

4. Spritze nach Ansprüche 1, bei der der Dehnungsabschnitt (13) als Ausnehmung gebildet ist.

5. Spritze nach einem der Ansprüche 1 bis 4, bei der die Anzahl der zweiten Rastelemente (12) wenigstens sechs beträgt, die in Umfangsrichtung verteilt sind und zwischen denen jeweils Dehnungsabschnitte (13) vorgesehen sind.

## Claims

1. Syringe having a cylinder (1) for accommodating a fluid medium and having, mounted at the outlet end, a closure cap (2) having a cover (4) provided with a sealable outlet (5),
the cylinder (1) having externally, on the portion arranged to be inserted into the cap (2), a first locking element (8), and the cap (2) having internally, on the corresponding portion, second locking elements (12) co-operating therewith,
the cap (2) having a plurality of second locking elements (12) arranged in a peripheral direction,
an expansion portion (13), which is elongate in the axial direction of the closure cap (2), being provided in the wall of the cap (2) between two locking elements (12) adjacent to one another, and
the expansion portion (13) extending, seen in the axial direction of the cap (2), beyond the extents, seen in the axial direction, of the second locking elements (12), **characterised in that**
that end of the expansion portion (13) which faces the open end (11) of the closure cap (2) is spaced apart from the open end (11) of the closure cap (2),
and the first locking element (8) is in the form of a nose, which projects out above the cylinder surface and which, on a portion (9) facing the outlet end, is bevelled off and which, on the second portion remote from the outlet end, has a shoulder (10) running virtually perpendicular to the outer surface of the cylinder.

2. Syringe having a cylinder (1) for accommodating a fluid medium and having, mounted at the outlet end, a closure cap (2) having a cover (4) provided with a sealable outlet (5),
the cylinder (1) having externally, on the portion arranged to be inserted into the cap (2), a first locking element (8), and the cap (2) having internally, on the corresponding portion, second locking elements (12) co-operating therewith,
the cap (2) having a plurality of second locking elements (12) arranged in a peripheral direction,
an expansion portion (13), which is elongate in the axial direction of the closure cap (2), being provided in the wall of the cap (2) between two locking elements (12) adjacent to one another, and
the expansion portion (13) extending, seen in the axial direction of the cap (2), beyond the extents, seen in the axial direction, of the second locking elements (12), **characterised in that**
that end of the expansion portion (13) which faces the open end (11) of the closure cap (2) is spaced apart from the open end (11) of the closure cap (2),
and the first locking element (8) is in the form of a recess (14) extending in a peripheral direction, which, on a portion remote from the outlet end, is bevelled off and which, on the second portion, which faces the outlet end, has a shoulder running virtually perpendicular to the outer surface of the cylinder.

3. Syringe according to claim 1 or 2, wherein the expansion portion (13) is in the form of a thinner wall portion.

4. Syringe according to claim 1, wherein the expansion portion (13) is in the form of a recess.

5. Syringe according to one of claims 1 to 4, wherein the number of second locking elements (12) is at least six, the locking elements being distributed in a peripheral direction and having respective expansion portions (13) provided between them.

## Revendications

1. Seringue avec un cylindre destiné à contenir un fluide (1) et un capuchon de fermeture (2) posé du côté de la sortie avec un couvercle (4) comportant une sortie (5) pouvant être obturée, le cylindre (1) comportant à l'extérieur un premier élément d'emboîtement (8) sur la partie pouvant être insérée dans le capuchon (2) et le capuchon (2) comportant à l'intérieur, sur la partie correspondante, des deuxièmes éléments d'emboîtement (12), le capuchon (2) comportant plusieurs deuxièmes éléments d'emboîtement (12) disposés sur la circonférence, une section de dilatation (13) oblongue dans le sens axial du capuchon de fermeture (2) étant disposée entre deux éléments d'emboîtement (12) voisins sur la paroi du capuchon de fermeture (2) et la section de dilatation (13), vue dans la direction axiale du capuchon (2), s'étendant dans la direction axiale des dilatations des deuxièmes éléments d'emboîtement (12), **caractérisée en ce que** l'extrémité de la section de dilatation (13) orientée vers l'extrémité ouverte (11) du capuchon de fermeture (2) est à une certaine distance de l'extrémité ouverte (11) du capuchon de fermeture (2) et **en ce que** le premier élément d'emboîtement (8) a la forme d'une saillie dépassant de la surface du cylindre, qui est chanfreinée sur la partie orientée vers l'extrémité du côté de la sortie (9) et qui comporte un épaulement (10) presque perpendiculaire à la surface du cylindre sur la seconde partie orientée à l'opposé de l'extrémité du côté de la sortie.

2. Seringue avec un cylindre destiné à contenir un fluide (1) et un capuchon de fermeture (2) posé du côté de la sortie avec un couvercle (4) comportant une sortie (5) pouvant être obturée, le cylindre (1) comportant à l'extérieur un premier élément d'emboîtement (8) sur la partie pouvant être insérée dans le capuchon (2) et le capuchon (2) comportant à l'intérieur, sur la partie correspondante, des deuxièmes éléments d'emboîtement (12), le capuchon (2) comportant plusieurs de deuxièmes éléments d'emboîtement (12) disposés sur la circonférence, une section de dilatation (13) oblongue dans le sens axial du capuchon de fermeture (2) étant prévue entre deux éléments d'emboîtement (12) voisins sur la paroi du capuchon de fermeture (2) et la section de dilatation (13), vue dans la direction axiale du capuchon (2), s'étendant dans la direction axiale des dilatations des deuxièmes éléments d'emboîtement (12), **caractérisée en ce que** l'extrémité de la section de dilatation (13) orientée vers l'extrémité ouverte (11) du capuchon de fermeture (2) est à une certaine distance de l'extrémité ouverte (11) du capuchon de fermeture (2) et **en ce que** le premier élément d'emboîtement (8) a la forme d'un évidement (14) s'étendant sur la circonférence, et est chanfreinée sur la partie opposée à l'extrémité du côté de la sortie et qui comporte, sur la partie orientée vers l'extrémité du côté de la sortie, un épaulement presque perpendiculaire à la surface du cylindre.

3. Seringue selon les revendications 1 ou 2, dans laquelle la section de dilatation (13) est une section de paroi d'épaisseur réduite.

4. Seringue selon la revendication 1, dans laquelle la section de dilatation (13) est un évidement.

5. Seringue selon l'une des revendications 1 à 4, dans laquelle le nombre des deuxièmes éléments d'emboîtement (12) est d'au moins six, répartis sur la circonférence et disposés entre les sections de dilatation (13).
